# EUROPÄISCHE PATENTSCHRIFT

(11) **EP 3 023 389 B1**
(45) Veröffentlichungstag und Bekanntmachung des Hinweises auf die Patenterteilung: **18.03.2020**
(21) Anmeldenummer: 15197731.1
(22) Anmeldetag: 05.07.2006
(51) Int. Cl.: C01B 21/24, B01J 19/12, C01B 21/28

(54) **VORRICHTUNG ZUR PHOTOLYTISCHEN ERZEUGUNG VON STICKSTOFFMONOXID**
DEVICE FOR PHOTOLYTIC PRODUCTION OF NITROGEN MONOXIDE
DISPOSITIF DE GENERATION PHOTOLYTIQUE DE MONOXYDE D'AZOTE

(43) Veröffentlichungstag der Anmeldung: 25.05.2016
(62) Teilanmeldung aus: 06013915.1
(73) Patentinhaber: BSN medical GmbH, 20253 Hamburg (DE)
(72) Erfinder: Suschek, Christoph V, 40764 Langenfeld (DE)
(74) Vertreter: Farago-Schauer, Peter Andreas

(56) Entgegenhaltungen:
- EP-B1- 0 560 928
- WO-A-94/20415
- US-A- 3 840 342
- US-A- 5 094 815
- US-A- 5 374 710

## Beschreibung

Die vorliegende Erfindung betrifft eine Vorrichtung zur photolytischen Erzeugung von Stickstoffmonoxid.

Stickstoffmonoxid (NO) ist ein biologisch wichtiges Molekül für die inner- und interzelluläre Reizleitung und für immunologische Reaktionen, welches normalerweise auch im menschlichen Körper gebildet wird. Die Entspannung von Blutgefässen wird von NO ebenso beeinflusst wie die Entspannung der Alveolen oder der glatten Muskulatur im gastrischen Bereich. Ein Mangel an NO kann umgekehrt dazu führen, dass die Relaxation der glatten Muskulatur unterbleibt und damit Konstriktionen auftreten. Eine solche Konstriktion äußert sich beispielsweise im bronchialen Bereich in Form von Asthma. Viele dieser Symptome lassen sich bekanntermaßen durch Inhalation NO-haltiger Gasgemische mit Konzentrationen im Bereich von 0,5 ppm bis 200 ppm beheben. Bei Neugeborenen mit Atemproblemen, beispielsweise im Falle eines Lungenhochdrucks, wenn das Kind einen Blausuchtsanfall hat, wird Stickstoffmonoxid als Medikament eingesetzt. Das Besondere daran ist, das NO so schnell wirkt wie kein anderes Medikament. Auch eine Herz-Lungen-Maschine kann nicht so schnell angeschlossen werden. Die Verwendung von Stickstoffmonoxid im pharmazeutischen Bereich ist beispielsweise in der EP 0 560 928 beschrieben.

Bisher wird ein für therapeutische Zwecke genutztes, NO-haltiges Inhalationsgas aus Gasflaschen (Industriegas) bereitgestellt, deren Lagerung und Handhabung in einer Klinik oder einer anderen Therapieeinrichtung aufgrund der erforderlichen Sicherheitsmaßnahmen aufwendig ist. Dies gilt insbesondere für eine mobile Vorrichtung. Zudem muss die Qualität des gelagerten Gases für medizinische Anwendungen hohen Anforderungen genügen, die den Aufwand für Herstellung und Lagerung weiter erhöhen. Schon eine geringe Verunreinigung des Gases führt nämlich zur Bildung unerwünschter und eventuell giftiger Nebenprodukte. Die europäischen Arzneimittel- und Gesundheitsbehörden haben demgemäß hohe Anforderungen an die Reinheit des zu verwendenden Stickstoffmonoxids gestellt.

Neben der Verwendung von "technischen" NO-Gasen für die medizinische Anwendung, gibt es Verfahren zur plasmachemischen Erzeugung von Stickstoffmonoxid. Aus den Druckschriften WO 95/07610 A , US 5,396,882 A und DE 198 23 748 A sind Verfahren zur plasmachemischen Erzeugung von NO bekannt, bei dem NO unter Einwirkung einer Glimm-, Funken- oder Lichtbogenentladung in einem Stickstoff (N₂) und Sauerstoff (O₂) enthaltenden Betriebsgas erzeugt wird. Eine Gasentladung der beschriebenen Art führt, wenn sie bei zu niedrigen Temperaturen durchgeführt wird (wie bei der Glimmentladung zu beobachten), zu einer geringen Effizienz der NO-Erzeugung in einem Gasgemisch. Zudem wird unter diesen Bedingungen bevorzugt das für Inhalationszwecke unerwünschte NO₂⁻Radikal (NO₂˙) erzeugt. Um das NO₂⁻Radikal NO₂˙ aus dem Inhalationsgas zu entfernen, ist der Einsatz einer aufwendigen Absorbertechnik erforderlich. Der Nachteil eines Absorbers liegt insbesondere darin, dass das Absorbermaterial häufig ausgetauscht oder wiederaufbereitet werden muss. Die im Vergleich zu einer Glimmentladung energiereichere Funken- oder Lichtbogenentladung bewirkt eine vergleichsweise starke Gasaufheizung, wodurch eine entsprechend effiziente NO-Erzeugung erzielt wird. Die insbesondere am Ansatzpunkt des Funkens hohe thermische Belastung der Elektroden führt jedoch in nachteiliger Weise zu einer starken Elektrodenerosion, d. h. einer fortschreitenden Zersetzung des Elektrodenmaterials. Aufgrund dieser Elektrodenerosion ist das Verfahren einerseits wartungsintensiv, da die Elektroden sehr verschleißanfällig sind. Anderseits muss verhindert werden, dass das im Inhalationsgas fein verteilte erodierte Elektrodenmaterial in die Atmungswege eines Patienten gelangt. Dies erfordert eine aufwendige Reinigung des Inhalationsgases.

Ferner ist auch eine Generierung von NO durch Photolyse möglich. Danach werden z.B. die in einer nitrithaltigen Lösung (z.B. Natriumnitrit) enthaltenen Nitrit-Ionen (NO₂⁻) mittels elektromagnetischer Strahlung (z.B. UVA-Strahlung mit Wellenlängen zwischen 320 und 440 nm) gespalten (Photolyse), wodurch NO generiert wird. Unter reduktiven Bedingungen bzw. unter Schutzgas (z.B. Stickstoff) verläuft der durch elektromagnetische Strahlung induzierte Zerfall von Nitrit über unterschiedliche, zum Teil auch parallele, thermodynamisch jedoch verschieden gewichtete Kanäle. Es kann angenommen werden, dass im Kanal 1 (Reaktionen 1 bis 5) UVA-Strahlung (mit einem Optimum bei 354 bis 366 nm) Nitrit zum Stickstoffmonoxidradikal (NO˙) und zum Sauerstoffradikalanion (O˙⁻) spaltet (Gleichung 1). Das zuletzt genannte Produkt initiiert im Folgenden in wässrigen Lösungen die Bildung des reaktiven Hydroxylradikals (OH˙) (Gleichung 2). Das Hydroxylradikal reagiert mit Nitrit, was zur Bildung des Nitrogendioxidradikals (NO₂˙) führt (Gleichung 3). Dieses kann mit Stickstoffmonoxid zu Dinitrogentrioxid (N₂O₃) weiterreagieren (Gleichung 4).

NO₂⁻+ hv -> NO˙ + O˙⁻ (1)

O˙⁻ + H₂O → OH˙ + OH⁻ (2)

NO₂⁻ + OH˙ → NO₂˙ + OH⁻ (3)

NO₂˙ + NO˙ → N₂O₃ (4)

N₂O₃ + H₂O → 2 NO₂⁻ + 2 H⁺ (5)

In Kanal 2 (Gleichungen 6-10) scheinen Hydroxylradikale unter den genannten Bedingungen keine Rolle zu spielen, jedoch werden ein "aquatisiertes" Elektron (e⁻_{aq}) sowie ein Stickstoffdioxidradikal gebildet (Gleichung 6). Das Elektron wird bei einem Überschuss an Nitrit auf dieses übertragen und das daraus resultierende Nitritanion (Gleichung 7) wird in Wasser zum NO-Radikal reduziert (Gleichung 8). Die folgenden Reaktionen in Gleichungen (9) und (10) entsprechen denen in Gleichungen (4) und (5). Die Gewichtung von Kanal 1 zum Kanal 2 bildet dabei ein Verhältnis von ca. 40:60.

NO₂⁻+ hv → NO₂˙ + e⁻_{aq} (6)

e⁻_{aq} + NO₂⁻ → NO₂⁻⁻ (7)

NO₂⁻⁻ + H₂O → NO˙ + 2 OH⁻ (8)

NO˙ + NO₂˙ → N₂O₃ (9)

N₂O₃ + H₂O → 2 NO₂⁻ + 2 H⁺ (10)

Wie aus den Reaktionen 1 bis 10 deutlich wird, wird der photolytische Zerfall von Nitrit von einer parallelen Produktion reaktiver und zytotoxischer, chemischer Spezies begleitet. Aus den Reaktionen in Gleichungen (4) und (9) wird zudem ersichtlich, dass NO₂-Radikale (NO₂˙⁻) mit dem in Gleichung (1) gebildeten NO rückreagieren können.

Der vorliegenden Erfindung liegt daher die Aufgabe zugrunde, eine verbesserte Vorrichtung zur Erzeugung von Stickstoffmonoxid in einem besonderen hohen Reinheitsgrad bereitzustellen, welches vorzugsweise im Wesentlichen frei von weiteren reaktiven oder toxischen, insbesondere zytotoxischen Spezies sein soll.

Darüber hinaus liegt der vorliegenden Erfindung die Aufgabe zugrunde, eine Vorrichtung bereitzustellen, mit welcher das Verfahren der NO-Generierung vorzugsweise besonders effektiv, preiswert, einfach und in einem breiten Verwendungsfeld durchführbar ist.

Gelöst wird diese Aufgabe durch eine Vorrichtung zur photolytischen Herstellung von Stickstoffmonoxid durch Spaltung einer Ausgangssubstanz gemäß dem Hauptanspruch.

Es wurde erkannt, dass durch den Einsatz von mindestens einem System, welches NO₂-Radikale oder Sauerstoffspezies abbaut oder neutralisiert, während der Generierung von Stickstoffmonoxid die Bildung der genannten reaktiven Zwischenprodukte des lichtinduzierten Nitritzerfalls (NO2˙, O˙⁻, OH˙, e⁻_{aq}) unterbunden wird oder deren Eliminierung erfolgt, während gleichzeitig die Generierung von Stickstoffmonoxid nicht gemindert wird. Somit wird erfindungsgemäß die Ausbeute frei verfügbarem NO erhöht und die Reinheit des Gases erhöht.

Der Anstieg der NO-Freisetzung sowie der hohe Reinheitsgrad beruht auf einer reaktionsbedingten Eliminierung der reaktiven Zwischenprodukte, z.B. gemäß den folgenden Reaktionen (11) bis (17).

N₂O₃ + RS⁻ → NO₂⁻ + RSNO (11)

RSNO + hv → NO˙ + RS˙ (12)

NO₂˙+ RS⁻ → NO₂⁻ + RS" (13)

NO˙ + RS → RSNO (14)

BA + OH˙ → BA-OH (15)

VitC+ NO₂˙ → NO₂⁻ + VitC˙⁻ (16)

Trol+ NO2˙ → NO₂⁻ + Trol˙⁻ (17)

(Abkürzungen: RS⁻, Thiol; GSNO, S-Nitros-Thiol; GS", Thioylradikal; BA, Benzoesäure; VitC, Vitamin C, Askorbat, Askorbinsäure; VitC", das Radikal von VitC; Trol, Trolox; Trol", das Radikal von Trolox).

Ein solches Verfahren ermöglicht durch die Präsenz dieser oder anderer funktionsgleicher Systeme während der Bildung von Stickstoffmonoxid eine hohe Ausbeute an Stickstoffmonoxid, während gleichzeitig die Bildung von unerwünschten (mehrfach) oxidierten Stickoxiden, insbesondere des NO₂˙, sowie von Hydroxylradikalen und reaktiven aquatisierten Elektronen effizient verhindert wird, oder diese Substanzen nach ihrer Entstehung eliminiert werden bzw. nur noch in so geringem Maße erzeugt werden können, dass sie in Lösung verbleiben und nicht in die Gasphase übertreten können. Somit können diese Substanzen z.B. keinen pathologisch relevanten Schaden in Folge der Inhalation der Inhalationsgase verursachen.

Als Systeme, welche reaktive Stickoxidspezies (z.B. Stickstoffdioxid (NO₂)-Radikale oder reaktive Sauerstoffspezies abbauen oder neutralisieren, werden vorzugsweise reaktive Sauerstoffspezies oder Stickoxidspezies (ROS oder RNS)-abbauende bzw. - neutralisierende Substanzen verwendet. Diese werden nachfolgend auch als Antioxidantien definiert und zusammengefasst. Weiter bevorzugt handelt es sich um Askorbinsäure, Askorbat, Vitamin E und seine Derivate, Thiole, Radikalfänger oder ROS- und RNS-abbauend Enzyme.

Es hat sich darüber hinaus herausgestellt, dass die Bindung oder Eliminierung der genannten reaktiven Zwischenprodukte des lichtinduzierten Nitritzerfalls (NO2˙, O˙⁻, OH˙, e⁻_{aq}) auch in einem neutralen pH-Bereich erfolgen kann, wobei aus Nitrit eine maximale NO-Freisetzung in einer maximalen Reinheit des NO-Gases erreicht werden kann.

Unter sauren Bedingungen (pH<7,0) wird in wässrigen Lösungen allerdings der "spontane" Nitritzerfall begünstigt. Entsprechend der Gleichungen 17-19 befindet sich das Nitritanion (NO₂⁻) in wässrigen Lösungen in einem Gleichgewicht mit seiner konjugierten Säure, der salpetrigen Säure (HNO₂). HNO₂ seinerseits befindet sich im Gleichgewicht mit Distickstofftrioxid (N₂O₃), welches spontan zu NO˙ und NO2˙ zerfällt.

NO₂⁻ + H⁺ ⇆ HNO₂ (18)

2 HNO₂ ⇆ N₂O₃ + H₂O (19)

N₂O₃ ⇆ NO˙ + NO₂˙ (20)

In einer Offenbarung der vorliegenden Anmeldung erfolgt daher die UVA-induzierte Generierung von Stickstoffmonoxid vorzugsweise in einem pH-Intervall von 0 bis 12, insbesondere von 1 bis 10, besonders bevorzugt von 1,5 bis 6, speziell von 2 bis 6, ganz speziell von 2,5 bis 4.

Je nach eingesetzter Nitrit- oder Antioxidantkonzentration sowie je nach Höhe des eingesetzten physikalischen Dekompensationsreizes, der zum Zerfall von Nitrit führt, kann durch das hier offenbarte Verfahren eine hohe Konzentration an Stickstoffmonoxid im Trägergas erreicht werden. So ist es durch das hier offenbarte Verfahren möglich, ausgehend von Nitrit, einen Stickstoffmonoxid-haltigen Trägergasstrom zu erzeugen, welcher vorzugsweise eine Konzentration an Stickstoffmonoxid von 1000 ppm oder höher aufweist. Dieser hochkonzentrierte Trägergasstrom übersteigt die für medizinische Anwendungen üblicherweise verträgliche NO-Konzentration von 5 bis zu 200 ppm um ein Mehrfaches.

Die Menge des generierten Stickstoffmonoxids in dem hier offenbarten Verfahren kann durch die verwendete Konzentration der das Stickstoffmonoxid freisetzenden Agenzien und durch die physikalische und/oder chemische Induktion, welche für die Freisetzung von Stickstoffmonoxid aus den Agenzien verantwortlich ist, gesteuert werden. Gegebenenfalls kann der durch das hier offenbarte Verfahren erzeugte Stickstoffoxid enthaltende Trägergasstrom auf eine entsprechend medizinisch anzuwendende Konzentration noch verdünnt werden.

Dabei werden im Rahmen der vorliegenden Erfindung unter dem Begriff "physikalische und/oder chemische Induktion" neben der Intensität der elektromagnetischen Strahlung sowie der Expositionsdauer, welcher die Reaktionslösung ausgesetzt wird, im Allgemeinen auch die Reaktionsparameter verstanden, welche einen Einfluss auf die Bildung von Stickstoffmonoxid an sich und auf die Konzentration an Stickstoffmonoxid haben. Hierzu zählen im Allgemeinen der pH-Wert der Reaktionslösung, der Redoxstatus der Reaktionslösung, die Temperatur der Reaktionslösung, die exponierte Bestrahlungsfläche, die Zeit der Einwirkung einer Induktionsgrößen auf die Stickstoffmonoxid freisetzenden Agenzien, die Strömungsgeschwindigkeit des Trägergases, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen. Insbesondere sind damit Chromophore und andere Substanzen gemeint, mit deren Hilfe z.B. auch elektromagnetische Strahlung außerhalb des UVA-Spektrumsbereiches in der Lage sein könnte aus den entsprechenden NO-bildenden Agenzien eine NO-Freisetzung zu ermöglichen.

So ist es beispielsweise bei konstant gehaltenen Induktionsgrößen durch den Einsatz variierender Konzentrationen der Stickstoffoxid freisetzenden Substanz(en) möglich, variierende Mengen an Stickstoffmonoxid freizusetzen.

Ferner ist es bei einer konstanten Konzentration der Stickstoffoxid freisetzenden Substanz(en) möglich, die Freisetzung von Stickstoffmonoxid durch Variation der Einstellparameter der jeweiligen Induktionsgrößen zu verändern. Bei einer konstant gehaltenen Induktionsgröße können daher in dem hier offenbarten Verfahren durch den Einsatz hoher Konzentrationen der NO-freisetzenden Substanzen hohe NO-Mengen freigesetzt werden und umgekehrt. Bei einer konstanten Konzentration der NO-freisetzenden Substanz kann die NO-Generierung durch Variation der Einstellparameter der jeweiligen Induktionsgrößen verändert werden. Die Einstellparameter können dabei alternativ oder gleichzeitig zur Regulation der NO-Erzeugung herangezogen werden. Insbesondere über die gleichzeitige Regelung der NO-Erzeugung über mehrere Einstellparameter kann das Verfahren vorteilhafterweise hinsichtlich der NO-Erzeugung sowie der Erzeugung unerwünschter Nebenprodukte optimiert werden.

Im Allgemeinen wird in dem hier offenbarten Verfahren die Konzentration an Stickstoffmonoxid und an dem Stickstoffdioxidradikal als entscheidende Messgrößen verwendet. Die Bestimmung der Messgrößen erfolgt dabei im Allgemeinen im rückverdünnten Betriebsgas, das als Endprodukt in dem hier offenbarten Verfahren erhalten wird. Stimmen diese Messgrößen nicht mehr mit den vorgegebenen Werten überein, kann durch Veränderung der Induktionsbedingungen eine Korrektur des Verfahrens erfolgen.

Die Substanz, welche zur Freisetzung von Stickstoffmonoxid verwendet und in dem hier offenbarten Verfahren verwendet wird, unterliegt grundsätzlich keiner Beschränkung, solange sie in der Lage ist, unter dem Einfluss von elektromagnetischer Strahlung Stickstoffmonoxid freizusetzen. Sie kann beispielsweise aus der Gruppe bestehend aus
(a) reinen Stoffen oder Stoffgemischen, welche unter dem Einfluss elektromagnetischer Strahlung Stickstoffmonoxid generieren;
(b) Stoffgemische, die zusätzlich zu den unter (a) genannten Stoffen oder Stoffgemischen Hilfssubstanzen enthalte, welche ausgewählt sind aus der Gruppe, bestehend aus Photoakzeptoren, Photoamplifier, Enzyme oder Katalysatoren, um spontan oder unter physikalischem oder chemischem Einfluss Stickstoffmonoxid generieren; und
(c) Stoffe oder Stoffgemische, die erst als Folge einer zuvor stattgefundenen chemischen Reaktion unter Zuhilfenahme der unter (a) genannten Stoffe und gegebenenfalls der unter (b) genannten Hilfssubstanzen unter dem Einfluss von elektromagnetischer Strahlung spontan Stickstoffmonoxid generieren,
ausgewählt werden.

Ferner können die unter (a) beschriebenen Substanzen zusätzlich durch Temperaturveränderungen und/oder Feuchtigkeitsveränderungen und/oder pH-Veränderungen ihrer Lösungen und/oder Veränderungen des Redoxstatus ihrer Lösungen Stickstoffmonoxid freisetzen.

In einer Ausführungsform der vorliegenden Erfindung erfolgt in der Vorrichtung die Freisetzung ausgehend von wässrigen Nitritlösungen. Dabei ist aus praktischen Gründen die Verwendung einer wässrigen Lösung von Natriumnitrit als Nitritquelle bevorzugt. Die wässrige Nitritlösung, insbesondere die wässrige Natriumnitritlösung, kann eine Konzentration an Nitrit von vorzugsweise 0,1 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweisen.

Die Dauer der Herstellung von Stickstoffmonoxid durch das hier offenbarte Verfahren unterliegt keiner besonderen Beschränkung und hängt von dem Gesamtbedarf an Stickstoffmonoxid und der erforderlichen Konzentration an Stickstoffmonoxid ab. Aus praktischen Gründen wird das hier offenbarte Verfahren jedoch in der Regel für einen Zeitraum von vorzugsweise 1 Minute bis 24 Stunden, insbesondere 1 Sekunde bis 12 Stunden, besonders bevorzugt 1 Sekunde bis 6 Stunden, speziell 2 Sekunden bis 2 Stunden, durchgeführt.

Die Art der Bestrahlung des nitrithaltigen Ausgangssubstrats ist dem auf dem vorliegenden Gebiet tätigen Fachmann an sich bekannt. Es kann jedwede elektromagnetische Strahlung verwendet werden, welche in der Lage ist, Nitrit unter Bildung von Stickstoffmonoxid zu zersetzten. Beispielsweise kann im Rahmen der vorliegenden Erfindung die Herstellung von Stickstoffmonoxid mittels photolytischer Spaltung unter Verwendung einer UVA-Strahlung mit Wellenlängen von beispielsweise 320 bis 440 nm erfolgen. Es kann jedoch auch elektromagnetische Strahlung jeder anderer Wellenlänge verwendet werden, die allein oder unter Zuhilfenahme chemischer, physikalischer oder biologischer Verfahren eine photolytische Spaltung von Nitrit induziert.

Die hier offenbarte Herstellung von Stickstoffmonoxid kann unter einer Schutzgasatmosphäre erfolgen, wobei als Schutzgas beispielsweise Stickstoff oder Argon verwendet werden kann. Darüber hinaus sind auch alle weiteren Schutzgase im Rahmen der vorliegenden Offenbarung verwendbar, solange sie sich gegenüber dem hier offenbarten Reaktionssystem innert verhalten.

Das erfindungsgemäß erhaltene Stickstoffmonoxid wird vorzugsweise mit einem Trägermittel vermischt und in dieser erhaltenen Form als Betriebsgas den gewünschten Anwendungen zugeführt.

Als Trägermedium für das erfindungsgemäß freigesetzte Stickstoffmonoxid werden vorzugsweise ungiftige gasförmige Substanzen oder deren Gemische verwendet. Beispiele für das Trägergas sind Edelgase, Stickstoff (N₂), sauerstoffhaltige Gase, wie Luft oder definierte synthetische Gas- oder Luftgemische. Weitere Beispiele für das Trägermedium sind Flüssigkeiten (z.B. Stickstoffmonoxidgas gesättigte Kultur- oder Infusions-Medien bzw. - Puffer, Serum, Blut Gele) oder feste Stoffe.

Gemäß der vorliegenden Erfindung werden bei der Vorrichtung Vorkehrungen getroffen, welche eine erneute Bildung des Stickstoffdioxidradikals in dem Betriebsgas vermeiden. Hierzu zählt beispielsweise eine Kühlungsvorrichtung, durch welche das Betriebsgas geleitet wird und durch welche eine unerwünschte Folgereaktion in dem mit Stickstoffmonoxid angereicherten Betriebsgas unterdrückt werden kann. Durch die Kühlungsvorrichtung wird beispielsweise eine Rückreaktion zu dem Stickstoffdioxidradikal im Betriebsgas unterdrückt. Eine weitere Möglichkeit, eine unerwünschte Folgereaktion in dem mit Stickstoffmonoxid angereicherten Betriebsgas, beispielsweise eine Rückreaktion zu dem Stickstoffdioxidradikal, zu unterdrücken, besteht ferner darin, durch Absorptionsvorrichtungen oder Katalysatoren, insbesondere zur Reduktion des Stickstoffdioxidradikals, dessen Bildung im Betriebsgas im Wesentlichen oder vollständig zu unterdrücken.

Insgesamt ist in einer besonderen Ausführungsform der erfindungsgemäßen Vorrichtung das NO-freisetzende Verfahren damit durch die folgenden Verfahrensschritte gekennzeichnet:
(1) Einfüllen von mindestens einer Ausgangssubstanz, welche zur Freisetzung von Stickstoffmonoxid geeignet ist, und mindestens eines Systems, welches Sauerstoffspezies abbaut oder neutralisiert, in eine Reaktionskammer;
(2) lichtinduzierter Zerfall der Ausgangssubstanz durch Bestrahlung der Mischung aus Ausgangssubstanz und dem mindestens einen System unter Freisetzung von Stickstoffmonoxid;
(3) Entfernen des in Schritt (2) gebildeten Stickstoffmonoxids aus der Reaktionskammer mittels eines Trägermediums unter Erhalt eines Betriebgases, welches Stickstoffmonoxid enthält.

Das durch die erfindungsgemäße Vorrichtung hergestellte Stickstoffmonoxid kann beispielsweise zu Inhalationszwecken eingesetzt werden. Weitere spezifische Anwendungsgebiete sind die Anregung des Stoffwechsels von Geweben durch äußerliche Anwendung, die strukturelle Modifikation organischer sowie anorganischer Flächen, die Sterilisation oder die Erzeugung von Zytotoxizität. Das durch das hier offenbarte Verfahren erzeugte Stickstoffmonoxid kann auch zur Begasung von Wunden, insbesondere zur Wundheilung, der Oberhaut, insbesondere zum Peeling, von Warzen und von Tumoren, insbesondere im Rahmen von nicht invasiven Maßnahmen, eingesetzt werden. Das Stickstoffmonoxid kann, wenn es in gesättigten Flüssigkeiten erzeugt wird, systemisch zur Behandlung von Bluthochdruck eingesetzt werden. Schließlich kann das Stickstoffmonoxid auch in mit Stickstoffmonoxid nitrosierte Träger erzeugt werden, welche spontan wieder NO freisetzen und z.B. atopisch im Rahmen dermatologischer Therapien eingesetzt werden. Das Stickstoffmonoxid kann auch zur Herstellung unterschiedlicher NO-bindender Substanzen (z.B. NO-Donoren) genutzt werden.

Das vorliegende offenbarte Verfahren weist eine Vielzahl an Vorteilen auf: Die Qualität eines gelagerten Gases für medizinische Anwendungen muss hohen Anforderungen genügen, die den Aufwand für Herstellung und Lagerung weiter erhöhen. Schon eine geringe Verunreinigung des Gases führt nämlich zur Bildung unerwünschter und eventuell giftiger Nebenprodukte. Die Entstehung dieser Nebenprodukte bei längerer Lagerung von Stickstoffmonoxid enthaltenden Gasflaschen sowie bei der plasmatechnischen Herstellung von Stickstoffmonoxid und die Entfernung dieser Radikale stellen einen großen technischen sowie finanziellen Nachteil dar. Die Vorteile des hier offenbarten Verfahrens zur Herstellung von Stickstoffmonoxid-Gasgemischen sind die Einfachheit der Herstellungsmethode des NOhaltigen Gases, der besonders hohe Reinheitsgrad des hergestellten NO-Gasgemisches, geringe Folgekosten und keine Lagerungskosten, die besonders einfache Handhabung der NO-Erzeugung sowie der Reinheitskontrolle, das unvergleichlich günstige Verhältnis der Herstellungskosten zu der Menge des hergestellten NO-Gases.

Die vorliegende Erfindung betrifft eine Vorrichtung, zur photolytischen Herstellung von Stickstoffmonoxid, welche zur Durchführung des oben beschriebenen Verfahrens geeignet ist..

Die erfindungsgemäße Vorrichtung zur Erzeugung von Stickstoffmonoxid ist dadurch gekennzeichnet, dass sie eine Reaktionskammer zur Erzeugung von Stickstoffmonoxid aufweist, die Reaktionskammer mindestens zwei Einlässe aufweist, wobei ein Einlass zur Zuführung des Ausgangsmaterials zur Bildung von Stickstoffmonoxid und ein Einlass zur Zuführung des Trägermaterials bestimmt ist, und die Reaktionskammer mindestens einen Auslass für das in der Reaktionskammer erzeugte Stickstoffmonoxid sowie das Trägermaterial aufweist.

Die in der Vorrichtung verwendete Reaktionskammer ist im Falle der photolytischen NO-Erzeugung vorzugsweise dadurch gekennzeichnet, dass sie aus einem Material aufgebaut ist, das die Eigenschaften der für eine Freisetzung von Stickstoffmonoxid notwendigen Energie einer elektromagnetischen Strahlungsquelle nicht beeinflusst oder aufgrund seiner Eigenschaften die für eine lichtinduzierte Freisetzung von Stickstoffmonoxid notwendigen Lichteigenschaften schafft, oder optimiert oder im Falle der pH-abhängigen NO-Generierung den pH-induzierten Nitritzerfall fördert und optimiert.

Die Lichtquelle in der erfindungsgemäßen Vorrichtung kann außerhalb und/oder innerhalb der Reaktionskammer vorzugsweise so angebracht sein, dass die Lichtdurchflutung des Reaktionskammerinhalts mitsamt den das Stickstoffmonoxid freisetzenden Reaktionssubstanzen im Sinne eines induzierten Stoffzerfalls bzw. einer Freisetzung von Stickstoffmonoxid optimal bzw. maximal ist.

Das Prinzip der hier vorgestellten neuen Vorrichtungen zur NO-Herstellung basiert darauf, dass in einer Reaktionskammer chemisch stabile oder stabilisierte, potentiell NOspeichernde und somit potentiell wieder NO-freisetzende Substanzen (z.B. organische oder anorganische Nitrite, S-, N- oder O-nitrosierte Verbindungen, NO-Metall-Verbindungen, NOchelatierende Substanzen), allein oder in unterschiedlichen Kombinationen, in reiner Form oder gelöst in unterschiedlichen Lösungsmitteln, in einer katalysierten oder nicht katalysierten, physikalisch gestarteten und/oder chemischen und/oder enzymatischen Reaktion NO freisetzen, welches mit Hilfe eines Trägergases zum Zwecke einer Anwendung abgeleitet werden kann. Dabei kann die NO-Freisetzung spontan verlaufen, durch physikalische, chemische oder biochemische Einflüsse induziert oder beschleunigt werden (z.B. durch eine Variation des pH und/oder durch eine physikalische, chemische, biochemische, biologischenzymatische Veränderung des Oxidations- oder Reduktionsstatus der NO-gespeicherten Substanz oder der Lösungen dieser Substanzen).

Die erfindungsgemäße Vorrichtung ist insbesondere zur Durchführung des oben beschriebenen Verfahrens geeignet.

In einer Ausführungsform der vorliegenden Erfindung erfolgt die photolytische Spaltung ausgehend von wässrigen Nitritlösungen, welche auch in dem hier offenbarten Verfahren verwendet werden können. Dabei ist aus praktischen Gründen die Verwendung einer wässrigen Lösung von Natriumnitrit als Nitritquelle bevorzugt. Die wässrige Nitritlösung, z.B. eine wässrige Natriumnitritlösung, kann eine Konzentration an Nitrit von vorzugsweise 0,1 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweisen.

Bei der photolytisch induzierten NO-Bildung können in der Reaktionskammer je nach gewünschter bzw. benötigter Höhe der Konzentration des NO-Gases sowie je nach gewünschter bzw. benötigter Dauer der NO-Freisetzung NO-generierende feste Stoffe in spezifischer Menge oder Lösungen mit Nitritsalzen oder den anderen oben erwähnten NO-generierenden Substanzen in spezifischen Konzentrationen verwendet werden. Hinsichtlich der Konzentration des NO-Gases, der benötigten Dauer der NO-Freisetzung und der spezifischen Konzentration der Nitritsalze wird auf obige Ausführungen verwiesen. Die Quelle zur Bildung von Stickstoffmonoxid kann der Reaktionskammer über eine Pumpe, welche gegebenenfalls über ein Zuflussventil geregelt ist, zugeführt werden.

Die Reaktionskammer der erfindungsgemäßen Vorrichtung wird vorzugsweise aus einem Material gebildet, das die Eigenschaften des für eine optimale NO-Generierung notwendigen Energie einer elektromagnetischen Strahlungsquelle nicht beeinflusst oder aufgrund seiner Eigenschaften die für eine lichtinduzierte NO-Generierung notwendigen Lichteigenschaften erst schafft oder optimiert. Sinnvollerweise ist die Lichtquelle außerhalb und/oder innerhalb der Reaktionskammer so angebracht, dass die Lichtdurchflutung des Reaktionskammerinhalts mitsamt den NO-freisetzenden Reaktionssubstanzen im Sinne eines induzierten Stoffzerfalls bzw. NO-Freisetzung maximal bzw. optimal ist. Die Reaktionskammer ist vorzugsweise dicht abgeschlossen und verfügt über eine Eintrittsöffnung, durch die ein Trägergas in die Kammer einströmen kann und den Kammerinhalt durchflutet sowie eine Austrittsöffnung, durch die das mit NO-Gas angereicherte Trägergas aus der Kammer wieder austritt.

Grundsätzlich unterliegt die in der erfindungsgemäßen Vorrichtung vorgesehene Reaktionskammer keiner weitergehenden Beschränkung. Die Reaktionskammer kann fest mit der medizinischen/kosmetischen Apparatur verbunden sein, sie kann jedoch auch austauschbar sein. Durch die Befüllung mit Agenzien in unterschiedlichen Kombinationen und Konzentrationen können die unterschiedlich bestückten Reaktionskammern über unterschiedliche NO-Freisetzungsmuster verfügen und somit unterschiedlichen Anwendungsanforderungen angepasst werden. Die Reaktionskammern können sich bezüglich ihrer Bestückung mit NO-generierenden Stoffen oder Lösungen unterscheiden, sodass sie sich durch die Länge der NO-Freisetzung sowie in der Höhe der freigesetzten NO-Menge unterscheiden und auf diese Weise charakterisiert werden können. Die NO-Gas-Freisetzungsmuster der jeweiligen Reaktionskammer können somit an den Bedarf der kosmetischen oder medizinischen Anwendung bzw. an die Fachkompetenz des Anwenders angepasst werden und sind in jedem Fall der angestrebten Anwendung optimal angepasst. Die Reaktionskammer kann fest in dem Gesamtgerät installiert sein und zum Zweck der NO-Generierung mit den entsprechenden Lösungen und/oder Chemikalien bestückt werden. Die Reaktionskammer kann jedoch auch ein austauschbares System darstellen, sodass sie erst vor dem Gebrauch des Gerätes in die Apparatur eingesetzt wird. Dabei kann die Reaktionskammer bereits mit den entsprechenden Chemikalien und Lösungen zur NO-Freisetzung befüllt sein oder sie wird erst kurz vor dem Einsatz in die Apparatur mit den entsprechenden Lösungen zur NO-Generierung bestückt und anschließend zum Zweck der NO-Generierung in die Apparatur eingesetzt.

Die Reaktionskammer kann auch ein Multikomponentensystem darstellen, bei dem alle für die NO-Gassynthese notwendigen Komponenten räumlich oder funktionell voneinander getrennt mit der Kammer assoziiert sind und erst kurz vor der gewünschten NO-Gassynthese durch Öffnung von Ventilen, Septen oder Trennwänden zusammentreffen und zur NO-Produktion vermischt werden können.

Die NO-Erzeugung wird vorzugsweise anhand der Manipulation der Einstellparameter geregelt werden. Als Einstellparameter gelten hier die eingesetzten Konzentration der NO-freisetzenden Agenzien, die elektromagnetische Strahlung und die Eigenschaften der weiteren physikalischen und/oder chemischen Induktionsgrößen, welche für die NO-Freisetzung aus den Agenzien verantwortlich sind. Als Induktionsgrößen einer NO-Freisetzung aus potentiell NO-generierenden Substanzen können in der Reaktionskammer einzeln oder in unterschiedlichen Kombinationen folgende Parameter variiert und genutzt werden: der pH-Wert der Reaktionslösung, der Redoxstatus der Reaktionslösung (Präsenz reduzierender oder oxidierender Stoffe), die Temperatur der Reaktionslösung, der atmosphärischer Druck in der Reaktionskammer, die Intensität der elektromagnetischen Strahlung und Expositionsdauer, welcher die Reaktionslösung ausgesetzt wird, die exponierte Bestrahlungsfläche, die Zeit der Einwirkung einer Induktionsgrößen auf die NO-freisetzenden Agenzien, die Strömungsgeschwindigkeit des Trägergases, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen. Insbesondere sind damit Chromophore und andere Substanzen gemeint, mit deren Hilfe z.B. auch elektromagnetische Strahlung außerhalb des UVA-Spektrumsbereiches in der Lage sein könnte, aus den entsprechenden NO-bildenden Agenzien eine NO-Freisetzung zu ermöglichen.

Bei einer beispielsweise konstant gehaltenen Induktionsgröße können durch den Einsatz variierender Konzentrationen der Stickstoffoxid freisetzenden Substanzen variierende Mengen an Stickstoffoxid erzeugt werden.

Ferner ist es bei einer konstanten Konzentration der Stickstoffoxid freisetzenden Substanz möglich, die Freisetzung von Stickstoffmonoxid durch Variation der Einstellparameter der jeweiligen Induktionsgröße zu verändern.

Bei einer konstant gehaltenen Induktionsgröße können daher in der erfindungsgemäßen Vorrichtung durch den Einsatz hoher Konzentrationen der NO-freisetzenden Substanzen hohe NO-Mengen freigesetzt werden und umgekehrt. Bei einer konstanten Konzentration der NO-freisetzenden Substanz, kann die NO-Generierung durch Variation der Einstellparameter der jeweiligen Induktionsgrößen verändert werden. Die Einstellparameter können dabei alternativ oder gleichzeitig zur Regulation der NO-Erzeugung herangezogen werden. Insbesondere über die gleichzeitige Regelung der NO-Erzeugung über mehrere Einstellparameter kann das Verfahren vorteilhafterweise hinsichtlich der NO-Erzeugung sowie der Erzeugung unerwünschter Nebenprodukte optimiert werden. Als Messgrößen können für die Regelung dabei die Konzentration an NO und an NO₂˙ herangezogen werden. Die Regelung kann beispielsweise mittels vorgegebener Kennlinien, die den Zusammenhang zwischen den Messgrößen und den Einstellgrößen herstellen, erfolgen. Die Messgrößen werden vorzugsweise im rückverdünnten Betriebsgas ermittelt, das als Endprodukt an einen Patienten oder Konsumenten aus der Apparatur ausgeleitet wird. Auf diese Weise kann die einwandfreie Durchführung des Verfahrens anhand der Messgrößen besonders zuverlässig kontrolliert werden. Ein Überschreiten vorgegebener kritischer Grenzwerte für die Messgrößen, insbesondere der Konzentration von NO oder von NO₂˙, wird vorzugsweise unmittelbar angezeigt, so dass die weitere Gaserzeugung gegebenenfalls angepasst oder gestoppt werden kann.

Die hochkonzentrierte NO-Erzeugung läuft mit einem besonders hohen Wirkungsgrad ab und ist besonders energiesparend. Zur Erreichung einer medizinisch verträglichen NO-Konzentration im Betriebsgas kann das NO-angereicherte Betriebsgas mit unbehandelten, NO-freien Gasen rückverdünnt werden.

. Erfindungsgemäß wird die Sicherstellung der maximalen Unterdrückung der NO₂˙-Existenz im auszuleitenden Gasgemisch durch die Verwendung von NO₂˙-spezifischen Absortionsvorrichtungen oder entsprechender Katalysatoren zur Reduktion des NO₂˙ erreicht.

Aufgrund der Möglichkeit, die Reaktionskammer in Form von Modulen mit fest eingestellten, konstanten und unveränderbaren Stellgrößen bezüglich der Dauer, Freisetzungsmenge sowie Freisetzungsprofil und Reinheitsgrad der NO-Freisetzung spezifisch und gezielt auf die gewünschte Anwendung hin zu gestalten, kann eventuell auf eine Regulation der erwähnten Stellgrößen verzichtet werden.

Als Trägermedien können Trägergase dienen. Als Trägergase des in der Rektionskammer gebildeten NO können alle ungiftigen gasförmigen Substanzen oder deren Gemische verwendet werden. Im Fall der Nutzung NO-haltiger Gasgemische zur äußerlichen Anwendung können als Trägergase inerte Gase wie z.B. die Edelgase oder Stickstoff (N₂), aber auch sauerstoffhaltige Gase wie Luft oder definierte synthetische Luftgemische verwendet werden. Im Fall der Nutzung NO-haltiger Gasgemische zum Zwecke einer Inhalationstherapie kann Stickstoff als Trägergas benutzt werden, welches unmittelbar vor der Lungeninhalation des Patienten, innerhalb oder außerhalb der beschriebenen Vorrichtung unter Verwendung einer Mischungsbatterie mit der gewünschten Menge reinen Sauerstoffs zu einem physiologisch verwendbaren atembaren Gasgemischt vermischt wird.

Je nach Anwendung können jedoch auch Flüssigkeiten, Gele oder feste Stoffe als Träger-, Bindungs- oder Transportmedien für das NO dienen. Es ist denkbar, mit NO-Gas gesättigte Flüssigkeiten wie Puffer, Lösungen, Medien, Serum oder Blut als Trägermedium zu verwenden und diese z.B. im Rahmen einer Therapie wieder systemisch in die Blutbahn einzuleiten oder NO-gesättigte, zähflüssige Trägermedien wie Gele atopisch zu Behandlung von Wunden zu verwenden. Das Trägermedium wird vorzugsweise separat, d.h. unabhängig von der Stickstoffmonoxid-Quelle, über einen eigenen Einlass in die Reaktionskammer geführt.

Die erfindungsgemäße Vorrichtung kann beispielsweise zur Erzeugung von NOhaltigen Gasgemische dienen, welche zu Inhalationszwecken eingesetzt werden. Je nach erzeugter NO-Menge bzw. je nach Spezifikation und Charakterisierung der Reaktionskammer kann es sich dabei um durch einen Arzt kontrollierte stationäre oder ambulante oder durch den Laien privat durchgeführte Inhalationen handeln. Der NO-Gasstrom kann zur Anregung des Stoffwechsels von Geweben durch äußerliche Anwendung, zur strukturellen Modifikation organischer sowie anorganischer Flächen, zur Sterilisation oder zur Erzeugung von Zytotoxizität genutzt werden. Im Bereich der Dermatologie könnte der erzeugte NO-Gasstrom zudem zur Begasung von Wunden (Wundheilung), der Oberhaut (Peeling), von Warzen sowie von Tumoren (nicht invasive Maßnahmen) verwendet werden. NO-gesättigte Flüssigkeiten können systemisch zur Behandlung von Bluthochdruck benutzt werden. Mit Hilfe von NO-Gas nitrosierte Träger können atopisch im Rahmen dermatologischer Therapien verwendet werden. Das durch das genannte Verfahren bzw. die beschriebene Vorrichtung erzeugte NO kann auch zur Herstellung unterschiedlicher NO-Speichernder Substanzen (z.B. NO-Donoren) verwendet werden.

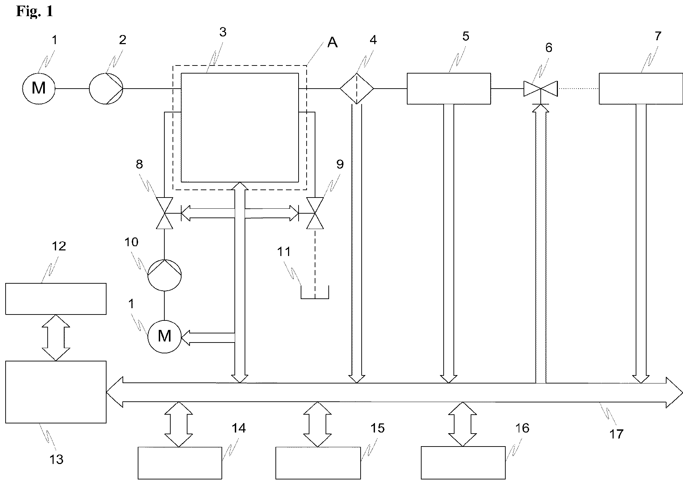

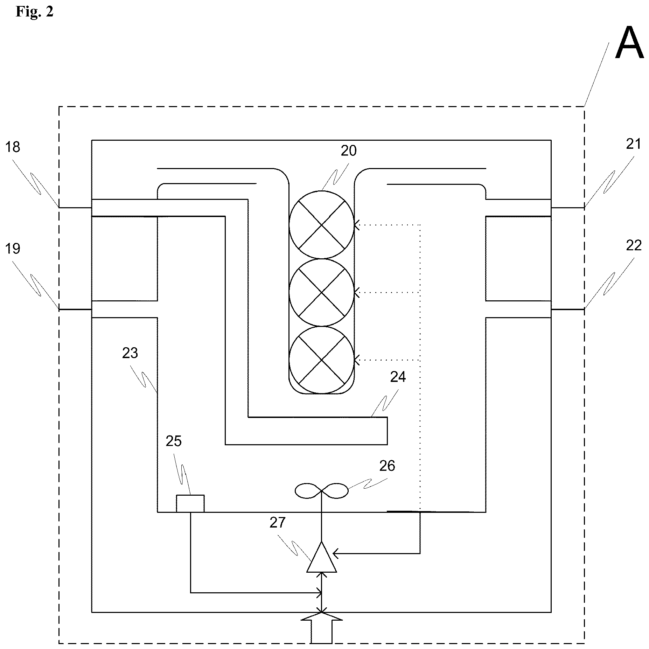

Im Folgenden wird zur Veranschaulichung eine Vorrichtung anhand der Figuren 1 und 2 beschrieben, wobei diese Vorrichtung nicht unter der vorliegenden Erfindung fällt.

Fig. 1 zeigt schematisch den Aufbau einer Vorrichtung zur Herstellung von Stickstoffmonoxid. Durch die mit dem Bezugszeichen (1) gekennzeichneten Pumpenmotoren wird einerseits ein Trägergas oder Druckluft (2) in eine Reaktionskammer (3) geführt. Durch das Zuflussventil (8) ist es möglich, den Zufluss von NO-freisetzenden Substanzen zu der Reaktionskammer (3) zu variieren, d.h. zu begrenzen oder zu erhöhen. In der Reaktionskammer (3) findet die Umsetzung unter Bildung von Stickstoffmonoxid statt. Aus der Reaktionskammer (3) kann durch das Abflussventil (9) Substrat entnommen werden. Das der Reaktionskammer (3) entnommene Stickstoffmonoxid-haltige Produkt wird anschließend einem Filter (4) für Stickstoffdioxid, einem Detektor (5) für Stickstoffmonoxid und für Stickstoffdioxid zugeführt. Der Gas-Strom, welcher der Reaktionskammer (3) entnommen und durch die Vorrichtungsbestandteile (21) geführt wird, wird anschließend über ein Sicherheitsventil (6) entnommen und seinem Ziel (7), beispielsweise einem Patienten, zugeführt. Durch den Detektor (5) können als Messgrößen die Konzentrationen an Stickstoffmonoxid und Stickstoffdioxid-Radikal bestimmt werden, so dass gegebenenfalls auf die Verfahrensführung Einfluss genommen werden kann.

Die vollständige Vorrichtung wird mittels einer CPU und einem Adress-, Daten- und Steuerbus gesteuert.

Fig. 2 zeigt schematisch den Aufbau der Reaktionskammer (3; A). Durch die Einleitung (18) wird das Trägergas eingeleitet und durch die Zuführung (19) wird das Nitrit in die Reaktionskammer geführt. Die Zuführung (18) ist mit einer Fritte (24) versehen. Durch den Mischer (26) mit einer Spannungsversorgung (27) wird das Reaktionsgemisch innig miteinander vermischt und mit einer entsprechenden UV-A-Lampe (20) bestrahlt.

Das resultierende Stickstoffmonoxid kann der Öffnung (21) entnommen werden. Durch die Messapparatur (25) kann der in der Reaktionskammer (3; A) herrschende Druck, die herrschende Temperatur, die verwendete Lichtwellenlänge und -intensität bestimmt werden. Die hierdurch erhaltenen Daten werden in die entsprechende CPU übertragen.

Legende zu den Fig. 1 und 2:
1: Pumpenmotor
2: Trägergas- oder Druckluftpumpe
3: Reaktionskammer
4: NO₂-Filter
5: NO-, NO₂-Detektor
6: Sicherheitsventil
7: Patient
8: Zuflussventil
9: Abflussventil
10: NO₂-Pumpe
11: Abfluss
12: Benutzerschnittstelle
13: CPU
14: Speicher
15: Display
16: Audio
17: Adress-, Daten- und Steuerbus
18: Trägergas- oder O₂-Zufluss
19: NO₂-Zufluss
20: Lampen (UV-A)
21: NO-Abfluss
22: NO₂-Abfluss
23: Reaktionskammer
24: Fritte
25: Messapparatur (Druck, Temperatur, Lichtwellenlänge und -intensität, Medium-Konzentration...)
26: Mischer
27: Energiequelle

Die Vorteile der hier beschriebenen Vorrichtung beziehen sich auf die vollkommen fehlenden Lagerungskosten größerer NO-Gas-Lagerbestände. Aufgrund des Modul-Charakters der unterschiedlichen Reaktionskammern kann eine vordefinierte, kontrollierte Herstellung in einem großen Konzentrationsintervall des NO erreicht werden. Je nach Spezifikation der Kammer kann das produzierte NO zur professionellen Anwendung oder zu einer durch den Laien durchführbaren Anwendung genutzt werden. Vor dem Vermischen der unterschiedlichen Substanzen einer Reaktionskammer besitzen die einzelnen Chemikalien eine sehr lange Lebensdauer. Sie verderben nicht oder nur sehr langsam. Eine bei Flaschenlagerung nicht zu unterschätzende Gefahr der Vergiftung durch ausströmendes NO-Gas oder NO₂-Radikalenstehung ist nahezu ausgeschlossen, da nur verhältnismäßig geringe NO-Konzentrationen produziert werden. Alle für die Herstellung des NO-Gasgemisches notwendigen Chemikalien, technischen Vorrichtungen und Steuerungseinheiten sind sehr preiswert und einfach zu handhaben.

## Patentansprüche

1. Vorrichtung zur photolytischen Herstellung von Stickstoffmonoxid, **dadurch gekennzeichnet, dass** sie eine Reaktionskammer zur Erzeugung von Stickstoffmonoxid und eine außerhalb und/oder innerhalb der Reaktionskammer angebrachte Lichtquelle aufweist, die Reaktionskammer mindestens zwei Einlässe aufweist, wobei ein Einlass zur Zuführung des Ausgangsmaterials zur Bildung von Stickstoffmonoxid und ein Einlass zur Zuführung des Trägermaterials bestimmt ist, die Reaktionskammer mindestens einen Auslass für das in der Reaktionskammer erzeugte mit Stickstoffmonoxid angereicherte Betriebsgas aufweist und die Vorrichtung mit einer Stickstoffdioxidradikal-spezifischen Absorptionsvorrichtung oder einem Katalysator zur Reduktion des Stickstoffdioxidradikals im NOangereicherten Betriebsgas versehen ist.

2. Vorrichtung nach Anspruch 1, **dadurch gekennzeichnet, dass** die Reaktionskammer aus einem Material aufgebaut ist, das die Eigenschaften des für eine optimale Freisetzung von Stickstoffmonoxid notwendige Energie einer elektromagnetischen Strahlungsquelle nicht beeinflusst oder aufgrund seiner Eigenschaften die für eine lichtinduzierte Freisetzung von Stickstoffmonoxid notwendigen Lichteigenschaften erst schafft oder optimiert oder im Falle der pH-abhängigen NO-Generierung den pH-induzierten Nitritzerfall fördert und optimiert.

3. Vorrichtung nach Anspruch 1 oder 2, **dadurch gekennzeichnet, dass** die Lichtquelle außerhalb und/oder innerhalb der Reaktionskammer so angebracht ist, dass die Lichtdurchflutung des Reaktionskammerinhalts mitsamt den das Stickstoffmonoxid freisetzenden Reaktionssubstanzen im Sinne eines induzierten Stoffzerfalls bzw. einer Freisetzung von Stickstoffmonoxid maximal ist.

4. Vorrichtung nach einem der Ansprüche 1 bis 3, **dadurch gekennzeichnet, dass** die Reaktionskammer ein offenes System darstellt oder dicht abgeschlossen ist.

5. Vorrichtung nach einem der Ansprüche 1 bis 4, **dadurch gekennzeichnet, dass** die Reaktionskammer mit der gesamten Vorrichtung zur Erzeugung von Stickstoffmonoxid austauschbar verbunden ist, sodass sie erst vor dem Gebrauch des Vorrichtung in die Apparatur eingesetzt werden kann.

6. Vorrichtung nach einem den Ansprüche 1 bis 5, **dadurch gekennzeichnet, dass** in einer Reaktionskammer chemisch stabile oder stabilisierte, potentiell NOspeichernde und somit potentiell wieder NO-freisetzende Substanzen vorliegen.

7. Vorrichtung nach Ansprüchen 1 bis 6, **dadurch gekennzeichnet, dass** in der Reaktionskammer eine wässrige Lösung von Natriumnitrit vorliegt.

8. Vorrichtung nach Ansprüchen 1 bis 7, **dadurch gekennzeichnet, dass** das Ausgangsmaterial zur Bildung von Stickstoffmonoxid der Reaktionskammer über eine Pumpe zuführbar ist.

9. Vorrichtung nach Ansprüchen 1 bis 8, **dadurch gekennzeichnet, dass** die Reaktionskammer der erfindungsgemäßen Vorrichtung vorzugsweise aus einem Material gebildet wird, das die Eigenschaften des für eine optimale NO-Generierung notwendigen Energie einer elektromagnetischen Strahlungsquelle nicht beeinflusst oder aufgrund seiner Eigenschaften die für eine lichtinduzierte NO-Generierung notwendigen Lichteigenschaften erst schafft oder optimiert.

10. Vorrichtung nach Ansprüchen 1 bis 9, **dadurch gekennzeichnet, dass** die Lichtquelle außerhalb und/oder innerhalb der Reaktionskammer so angebracht ist, dass die Lichtdurchflutung des Reaktionskammerinhalts mitsamt den NO-freisetzenden Reaktionssubstanzen im Sinne eines induzierten Stoffzerfalls bzw. NO-Freisetzung maximal bzw. optimal ist.

11. Vorrichtung nach einem der Ansprüche 1 bis 10, **dadurch gekennzeichnet, dass** das Betriebsgas, welches das erzeugte Stickstoffmonoxid enthält, durch eine Kühlungsvorrichtung ableitbar ist.

## Claims

1. Device for photolytic preparation of nitrogen monoxide, **characterised in that** it comprises a reaction chamber for generating nitrogen monoxide and a light source that is attached outside and/or inside the reaction chamber, the reaction chamber comprises at least two inlets, wherein one inlet is intended for supplying the starting material for forming nitrogen monoxide, and one inlet is intended for supplying the carrier material, the reaction chamber comprises at least one outlet for the nitrogen monoxide-enriched operating gas generated in the reaction chamber, and the device is provided with a nitrogen dioxide radical-specific absorption device or a catalyst for reducing the nitrogen dioxide radical in the NO-enriched operating gas.

2. Device according to claim 1, **characterised in that** the reaction chamber is constructed from a material that does not influence the properties of the energy of an electromagnetic radiation source required for optimal release of nitrogen monoxide or, owing to the properties thereof, or originally provides or optimises the light properties required for light-induced release of nitrogen monoxide, or, in the case of the pH-dependent NO generation, promotes and optimises the pH-induced nitrite decomposition.

3. Device according to either claim 1 or claim 2, **characterised in that** the light source is attached outside and/or inside the reaction chamber, such that the light flooding of the reaction chamber content, together with the reaction substances that release the nitrogen monoxide is at a maximum, within the meaning of induced substance decomposition or release of nitrogen monoxide.

4. Device according to any of claims 1 to 3, **characterised in that** the reaction chamber represents an open system, or is closed in a sealed manner.

5. Device according to any of claims 1 to 4, **characterised in that** the reaction chamber is exchangeably connected to the overall device for generating nitrogen monoxide, such that it can be inserted into the apparatus just before the device is used.

6. Device according to any of claims 1 to 5, **characterised in that** substances that are chemically stable or stabilised and that potentially store NO and thus potentially release NO again, are provided in a reaction chamber.

7. Device according to claims 1 to 6, **characterised in that** an aqueous solution of sodium nitrite is provided in the reaction chamber.

8. Device according to claims 1 to 7, **characterised in that** the starting material for forming the nitrogen monoxide can be supplied to the reaction chamber via a pump.

9. Device according to claims 1 to 8, **characterised in that** the reaction chamber of the device according to the invention is preferably formed of a material which does not influence the properties of the energy of an electromagnetic radiation source required for optimal NO generation, or which, owing to the properties thereof, originally provides or optimises the light properties required for light-induced NO generation.

10. Device according to claims 1 to 9, **characterised in that** the light source is attached outside and/or inside the reaction chamber, such that the light flooding of the reaction chamber content, together with the NO-releasing reaction substances is at a maximum or is optimal, within the meaning of induced substance decomposition or release of nitrogen monoxide.

11. Device according to any of claims 1 to 10, **characterised in that** the operating gas which contains the generated nitrogen monoxide can be conducted away via a cooling device.

## Revendications

1. Dispositif de fabrication photolytique de monoxyde d'azote, **caractérisé en ce qu'**il présente une chambre de réaction servant à produire du monoxyde d'azote et une source de lumière installée à l'extérieur et/ou à l'intérieur de la chambre de réaction, la chambre de réaction présente au moins deux entrées, dans lequel une entrée se destine à amener le matériau de départ pour obtenir du monoxyde d'azote et une entrée se destine à amener le matériau de support, la chambre de réaction présente au moins une sortie pour le gaz de fonctionnement enrichi en monoxyde d'azote, produit dans la chambre de réaction et le dispositif est pourvu d'un dispositif d'absorption spécifique au radical de dioxyde d'azote ou d'un catalyseur servant à la réduction du radical de dioxyde d'azote dans le gaz de fonctionnement enrichi en NO.

2. Dispositif selon la revendication 1, **caractérisé en ce que** la chambre de réaction est élaborée à partir d'un matériau, qui n'a pas d'incidence sur les propriétés de l'énergie, requise pour une libération optimale de monoxyde d'azote, d'une source de rayonnement électromagnétique ou crée ou optimise, du fait de ses propriétés, les propriétés de lumière requises pour une libération induite par lumière de monoxyde d'azote ou favorise ou optimise, dans le cas de la génération de NO en fonction du pH, la décomposition de nitrite induite par le pH.

3. Dispositif selon la revendication 1 ou 2, **caractérisé en ce que** la source de lumière est installée à l'extérieur et/ou à l'intérieur de la chambre de réaction de telle sorte que le passage de lumière du contenu de chambre de réaction y compris les substances de réaction libérant le monoxyde d'azote est maximal au sens d'une décomposition de matière induite ou d'une libération de monoxyde d'azote.

4. Dispositif selon l'une quelconque des revendications 1 à 3, **caractérisé en ce que** la chambre de réaction constitue un système ouvert ou est fermée de manière hermétiquement étanche.

5. Dispositif selon l'une quelconque des revendications 1 à 4, **caractérisé en ce que** la chambre de réaction est reliée à l'ensemble du dispositif de manière interchangeable pour produire du monoxyde d'azote de sorte qu'elle peut être insérée dans l'appareil seulement avant l'utilisation du dispositif.

6. Dispositif selon l'une quelconque des revendications 1 à 5, **caractérisé en ce que** des substances chimiquement stables ou stabilisées, stockant potentiellement du NO et libérant du NO ainsi potentiellement à nouveau sont présentes dans une chambre de réaction.

7. Dispositif selon les revendications 1 à 6, **caractérisé en ce qu'**une solution aqueuse de nitrite de sodium est présente dans la chambre de réaction.

8. Dispositif selon les revendications 1 à 7, **caractérisé en ce que** le matériau de départ peut être amené à la chambre de réaction par l'intermédiaire d'une pompe pour obtenir du monoxyde d'azote.

9. Dispositif selon les revendications 1 à 8, **caractérisé en ce que** la chambre de réaction du dispositif selon l'invention est formée de préférence à partir d'un matériau, qui n'a pas d'incidence sur les propriétés de l'énergie, requise pour une génération optimale de NO, d'une source de rayonnement électromagnétique ou crée ou optimise du fait de ses propriétés les propriétés de lumière requises pour une génération de NO induite par lumière.

10. Dispositif selon les revendications 1 à 9, **caractérisé en ce que** la source de lumière est installée à l'extérieur et/ou à l'intérieur de la chambre de réaction de telle sorte que le passage de lumière du contenu de chambre de réaction y compris des substances de réaction de libération de NO est maximal ou optimal au sens d'une décomposition de matière ou de libération de NO induite.

11. Dispositif selon l'une quelconque des revendications 1 à 10, **caractérisé en ce que** le gaz de fonctionnement, lequel contient le monoxyde d'azote produit, peut être dévié par un dispositif de refroidissement.
